# EUROPEAN PATENT APPLICATION

(11) **EP 1 813 939 A1**
(43) Date of publication of application: **01.08.2007**
(21) Application number: 07250302.2
(22) Date of filing: 25.01.2007
(51) Int. Cl.: G01N 31/22

(54) **Ammonia detection device and method**

(30) Priority: 25.01.2006 US 761922 P
(71) Applicant: Virbac Corporation, Fort Worth, TX 76137 (US)
(72) Inventor: Kopelman, Roni Aron, Seattle, WA 98105 (US); Jamieson, Stuart, Seattle, WA 98105 (US); Thurma, John, Seattle, WA 98105 (US)
(74) Representative: Lockey, Robert Alexander

(57) **Abstract**

A reusable device for continuously monitoring gaseous ammonia concentration in a surrounding environment comprising a plurality of layers, including an active layer in which a dye (e.g., an indicator dye useful for measurement of pH) is immobilized to a membrane (e.g., polyamide membrane), and a hydrophobic filter layer impermeable to liquids and capable of permitting contact between the active membrane and dissolved gaseous ammonia.

## Description

### BACKGROUND OF THE INVENTION

### 1. Related Applications

This non-provisional patent application claims the benefit of U.S. Provisional Application Serial No. 60/761,922, filed January 25, 2006, titled "Ammonia Detection Device and Related Methods," which is incorporated by reference in its entirety.

### 2. Field of the Invention

This invention relates generally to a device and method for detecting and continuously monitoring dissolved ammonia concentration in various environments.

### 3. Description of Related Art

Environmental monitoring is a critical component of current global economics, with impacts in national security, commerce, pollution control, medical device functionality, and countless diagnostic applications. Hazardous material screening garners extreme attention, especially in light of today's heightened security concerns. As a result, inexpensive and accurate environmental monitors are highly sought after inventions.

The environmental media in which the monitoring is to take place is also extremely varied, consisting of atmospheric, aquatic, chemical, biological, and many other generic media. Consistent throughout the different applications and wide variety of potential environmental media, is the need for alarm systems that sense the presence of harmful compounds. Visual sensors, which respond optically to a target stimulus, are some of the most common, well-understood and functional types of monitoring systems.

Ammonia gas (NH₃) is a well known and somewhat universal chemical hazard. This chemical is acknowledged as a severe health hazard acting as both a poison and severe corrosive material. The Occupational Safety and Health Administration (OSHA) has determined the Permissible Exposure Limit (PEL) of ammonia to be 50 ppm (time weighted average or TWA) and the ACGIH Threshold Limit Value (TLV) at 25 ppm (time weighted average or TWA), 35 ppm (short term exposure limit or STEL).

Ammonia is also a known toxic chemical to fish species. As a byproduct of nitrogenous waste, ammonia presence is an inevitable reality for fish. The buildup and presence of ammonia is an especially troublesome aspect of ornamental fish keeping. Home and commercial aquariums are closed, miniature eco-systems in which a properly "cycled" tank will drastically reduce ammonia levels to negligible amounts. Nevertheless, a vast majority of hobbyists in the United States remain relatively unaware of the toxic effects of even very small concentrations of ammonia in their tanks. As a result, poor management of hobby tanks leads to high death rates for ornamental fish as ammonia presence persists as a serious problem in the hobby.

Currently, there are several commonly accepted methods for testing ammonia concentrations in aquatic environments:

*The Solution Method:* This colorimetric method is the most inexpensive option available. Three common techniques include the Nessler, Phenate, and titrimetric methods. Each of these methods involves removing a sample from the environment, adding chemicals to the sample, and allowing an indicator color to develop over a prescribed length of time. The reactions, however, are sensitive to many interferences and short or long color development periods, all leading to inaccurate results. Furthermore, the tests are laborious, require handling of dangerous chemicals (the Phenate method requires phenol or a phenol derivative and hypochlorite, and the Nessler method requires a mercury based compound), and the solutions degrade over time, necessitating either repurchase or additional preparation to make fresh solutions. Also, the Nessler and Phenate methods are not reversible, thus precluding continuous ammonia monitoring.

*The Dip-Strip Method:* This method utilizes a small reaction pad that is dipped into the sample environment, removed, and allowed to develop over the course of a specified time. Typically, a regent system or pH dye is contained within the pad, thus giving either a direct or indirect determination of ammonia concentration. The lack of true immobilization of the reagent system onto the pad makes this method dangerous as a potential contaminant of the aquatic environment. Furthermore, the reaction is irreversible, preventing this method from providing a continuous sensing method.

*Ion Sensitive Electrode Method:* This is typically the most expensive method and involves a complex electronic reference electrode and reference solution, separated from the sample environment via a hydrophobic gas permeable membrane. When the device is submerged into a basic sample solution, the free NH₃ gas from the sample diffuses across the hydrophobic membrane and reacts with the reference solution, which is read by the electronic equipment. This method requires at least daily calibration and routine maintenance of the probe and replacement of the hydrophobic membrane making it very expensive and laborious.

There are several other examples of ammonia sensitivity testing, including flow injection analysis in which a stream of pH dye is separated from a target stream containing dissolved ammonia by a hydrophobic membrane. The pH stream responds to the diffusing NH₃ by converting to the color attributed to higher pH values, which is then sensed by a spectrophotometer to provide accurate absorbance values and corresponding NH₃ values. This method requires calibration and routine maintenance of the electronic devices, making it expensive and laborious. Other methods immobilize pH dyes directly into hydrophobic materials, thus precluding contact of the pH dye with an aqueous solution but allowing diffusion of NH₃ into the pores of the material in order to contact the pH dye. These methods may result excessive amounts of dye bleeding or leaking from the hydrophobic materials, and do not allow for easy visualization by the user of the color change in the pH dye, due to the dye being embedded within the structure of the hydrophobic material.

### BRIEF SUMMARY OF THE INVENTION

The present invention relates to devices, systems, and methods for quickly and easily measuring and monitoring ammonia concentrations (e.g., gaseous ammonia concentrations) in a variety of aquatic and other environments.

In one aspect, the invention includes a reusable device for continuously monitoring gaseous ammonia concentrations comprising a plurality of layers, including an first active layer in which a dye (e.g., an indicator dye useful for measurement of pH) is immobilized to a membrane (e.g., a polyamide membrane), and a second hydrophobic filter layer capable of permitting contact between the active membrane and dissolved gaseous ammonia.

An embodiment of the present invention provides a reusable device for continuously monitoring the gaseous ammonia (NH₃) concentration in a surrounding environment. The device preferably has a first layer comprising a sensor membrane. An indicator dye is immobilized in the sensor membrane, wherein the indicator dye changes color corresponding to the gaseous ammonia concentration. The device also preferably has a second layer comprising a hydrophobic filter membrane permeable to gaseous ammonia, such that gaseous ammonia can diffuse through filter the membrane and contact the sensor membrane. The hydrophobic filter membrane preferably physically separates the sensor membrane from the surrounding environment. The device is fully immersible in an aqueous environment, if desired by the user.

In an aspect of the invention, the active membrane preferably has a plurality of sides, and can be contacted on one side by the hydrophobic filter membrane, and contacted on another side by a hydrophobic, optically transparent material. To the extent the hydrophobic filter membrane is a transparent composition, the hydrophobic optically transparent material can also be made from the transparent composition in an embodiment of the invention.

The hydrophobic, optically transparent material can include a color reference chart with a plurality of colors displayed thereon, wherein each color corresponds to a reference gaseous ammonia concentration value. The colors will preferably be in a range of shades from yellow to green to blue to indicate the level of gaseous ammonia concentration in the surrounding environment. In an embodiment of the invention, the color reference chart forms part of the device, the device being immersible in the aqueous solution. To the extent the hydrophobic filter membrane is a transparent composition, the color chart can be positioned on or adjacent to the hydrophobic filter membrane.

The color change in the active membrane can be viewed through the optically transparent material in an embodiment of the invention. It is not required that the optically transparent material allow for diffusion of the ammonia gas, as this is accomplished by the filter membrane. The optically transparent membrane should, however, preferably be hydrophobic so as to prevent water and contaminants to contact the sensor membrane.

In an aspect of the invention, the sensor membrane is a polyamide membrane. Also, the sensor membrane can be a positively charged, negatively charged or neutral membrane. The indicator dye can comprise one or more dyes from the group consisting of: bromophenol blue, congo red, methyl orange, resorcin blue, alizarin, methyl red, bromoceresol purple, chrophenol red, bromothymol blue, phenol red, litmus, neutral red, tumaric curcumin, and phenolphthalein. The sensor membrane can preferably change color in response to gaseous ammonia concentration. In an embodiment of the invention, the color change is reversible, such that subsequent increases or decreases in ammonia concentration in the surrounding environment over a period of time are viewable via the device. In a preferred embodiment, this also permits the device to be reusuable.

The surrounding environment for the device is preferably the aqueous environment, whereby the hydrophobic filter membrane of the device substantially prevents water, dissolved solids and other contaminants from contacting the sensor membrane. In a particularly preferred embodiment, the hydrophobic filter membrane is also effective to prevent ammonium ions from contacting the sensor membrane. The hydrophobic filter membrane can include one or more compounds from the group consisting of polytetrafluoroethylene, polypropylene, polyvinylidene fluoride, polyvinyl chloride, copolymers and blends of these compounds. The hydrophobic filter membrane preferably contacts and completely covers at least a side of the active membrane in an embodiment of the invention. Alternatively, there can be one or more layers between the filter membrane and the active membrane such that the filter membrane does not contact the active membrane.

An embodiment of the present invention provides a method for detecting the gaseous ammonia (NH₃) concentration in an aqueous environment. The method preferably comprises providing a reusuable device having at least a first layer and a second layer. The first layer has a sensor membrane with an indicator dye immobilized therein, wherein the indicator dye changes color corresponding to the gaseous ammonia concentration. The second layer preferably includes a hydrophobic filter membrane permeable to gaseous ammonia such that gaseous ammonia can diffuse therethrough and contact the sensor membrane. The device is immersed in an aqueous environment; and a user optically views the color change in the sensor membrane upon the gaseous ammonia contacting the sensor membrane.

### BRIEF DESCRIPTION OF THE DRAWINGS

In the drawings:

FIG. 1 is a top view of the chemical structure of bromothymol blue as utilized in an embodiment of the present invention;

FIG. 2 is a top view of a mechanism of phenolic proton abstraction from BTB by an ammonia molecule as utilized in an embodiment of the present invention;

FIG. 3 is a pair of graphs indicating the relationship between color (hue) and ammonia concentration over time, demonstrating reversibility of colorimetry, according to an embodiment of the present invention;

FIG. 4 is a graph indicating the relationship between color (hue) and ammonia concentration according to an embodiment of the present invention;

FIG. 5 is a perspective multi-layered view of a functional ammonia detection device according to an embodiment of the present invention; and

FIG. 6 is a view of a design model of an ammonia detection device having a color reference chart thereon according to an embodiment of the present invention.

While the invention will be described in connection with the preferred embodiments, it will be understood that it is not intended to limit the invention to that embodiment. On the contrary, it is intended to cover all alternatives, modifications, and equivalents, as may be included within the spirit and the scope of the invention as defined by the appended claims.

### DETAILED DESCRIPTION OF THE INVENTION

The present invention includes devices, systems, and methods for real-time, continuous detection (e.g., colorimetric detection) of gaseous ammonia (NH₃). In an embodiment of the invention, the device includes a solid-state membrane material composed of multiple layers, including a first layer comprising an active membrane in which an indicator dye is immobilized to a polyamide or like membrane, and a second layer comprising a hydrophobic filter membrane. Upon submersion in an aqueous solution, gaseous ammonia diffuses through the filter membrane and contacts the active membrane of the device, causing the device to change color according to the concentration of dissolved ammonia.

The device can be used, for example, to determine ammonia levels in ornamental aquariums (e.g., 0-0.5 ppm NH₃). The device can also be used in other ammonia containing aqueous environments, and therefore the technology can be specifically tailored to suit the concentration range and conditions typical in these particular environments. That is, the sensitivity, reaction time, and lifetime may all be tailored to suit a specific function by carefully selecting the materials used for construction, as outlined in the below illustrative example.

### Illustrative Example

In an embodiment of the present invention, the device functions due, in part, to several sequential events occurring within the device. The device typically comprises two layers, an active membrane layer in which an indicator dye is immobilized to a polyamide or like membrane and a hydrophobic filter membrane layer.

In an embodiment of the present invention, the active membrane layer or "sensing" layer contains a pH dye (preferably bromothymol blue or "BTB") immobilized, that is, permanently bound onto and into the layer through electrostatic interactions in addition to hydrogen bonding and attractive van der Waals forces. In one embodiment, the layer is composed of a positively charged nylon membrane, although negatively charged or neutral membranes may also be utilized. In general, indicator dyes, membranes, and dye-containing membranes suitable for use in the present invention and methods of fabrication thereof are described, for example, in commonly owned U.S. Patent Application No. 11/127,849, filed May 12, 2005, titled "Reusable pH Sensor Device and Related Methods", the entire content of which is incorporated herein by reference, for all purposes.

The active membrane layer serves as the surface on which the ammonia gas (NH₃) contacts the pH dye and reacts with the dye in the following manner:

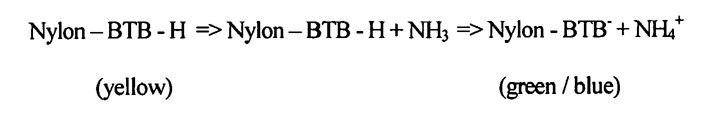

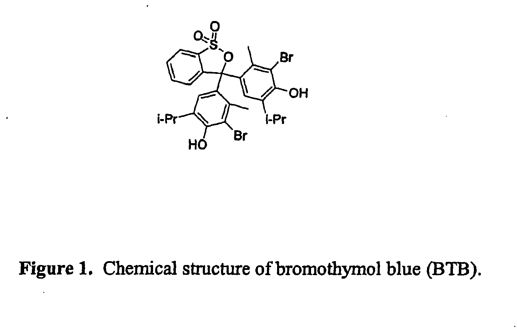

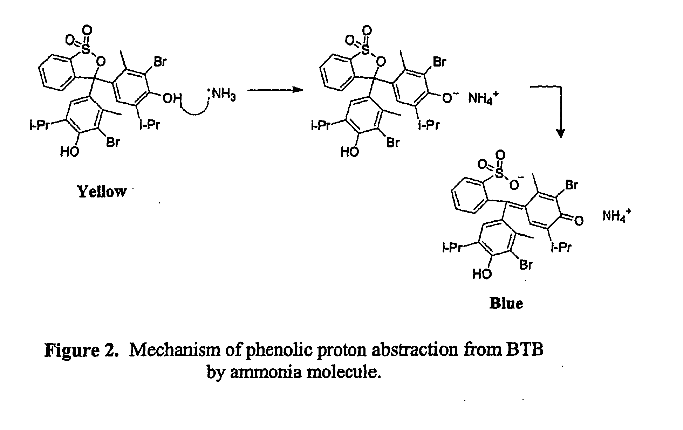

The color development of the active membrane is directly proportional to ammonia gas concentration, as will be discussed in more detail below. In general, the bromothymol blue adopts a yellow color with a slight hint of green in the fully protonated state, which corresponds to the chemical structure set forth in FIG. 1. Upon contact with gaseous ammonia, a phenol proton is removed by the ammonia molecule according to the process known in the art, as set forth in FIG. 2.

The fully protonated (yellow) form of BTB does not have a fully delocalized electronic structure as the methane carbon connecting the two phenolic moieties maintains *sp*³ hybridization. Upon deprotonation by the ammonia (NH₃) molecule, the dominant resonance structure becomes that of the structure depicted for the "Blue" state shown in FIG. 2. The deprotonation occurs due to the localized basic environment that the dye molecule is in as a result of the NH₃ presence and the ammonia's basic and nucleophilic nature. The consequence of this reaction is the quinone-like electronic structure which allows for complete delocalization of the π electrons due to the fully conjugated chemical nature and lack of any interrupting *sp*³ hybridized carbon atoms.

The color observed on the active membrane is directly related to the electronic structure of the molecule. The fully delocalized structure of BTB is known to be responsible for the deep blue color observed at higher pH values, which corresponds to the deprotonated/high NH₃ value structure. The chemistry and associated optical properties of triphenyhnethane (also known as sulphonphthalein) dyes, as are utilized in an embodiment of the invention, are well known and have been documented extensively in the prior art.

A high value of NH₃ is analogous to a high pH value as the chemical reaction is equivalent, resulting in a system in which high ammonia values leads to increasingly blue color development.

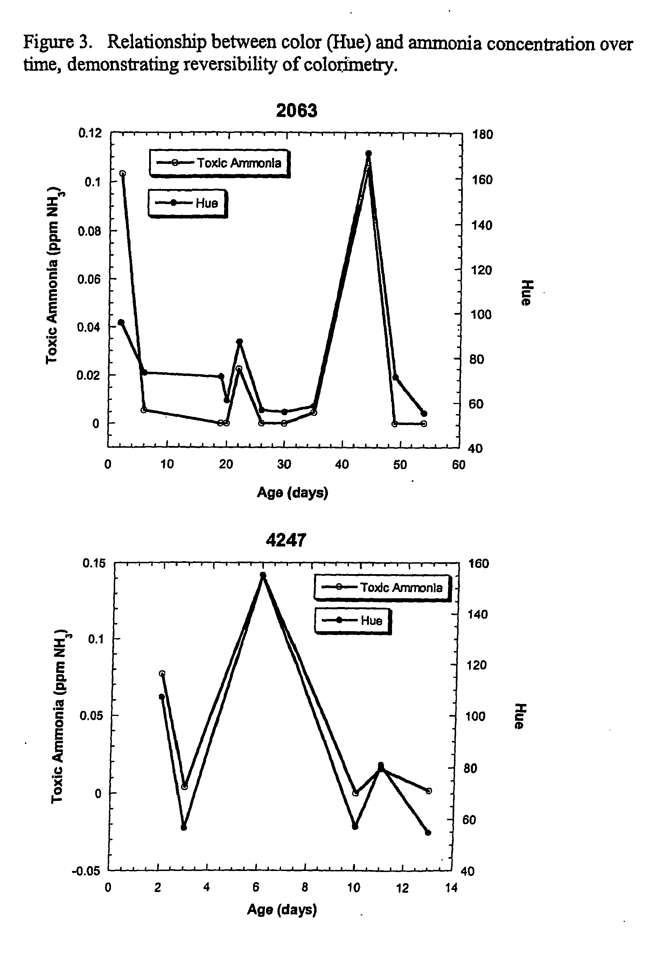

Reversibility, that is, the ability to achieve accurate continuous measurement, is also a feature of the device according to an embodiment of the invention. The sensor membrane can preferably change color in response to gaseous ammonia concentration. The color change is preferably reversible, such that subsequent increases or decreases in ammonia concentration in the surrounding environment over a period of time are viewable via the device as the device remains fully or partially submerged in the surrounding environment for a prolonged period. FIG. 3 shows an illustration of the reversibility of the color reaction over time in reference to various toxic ammonia concentrations.

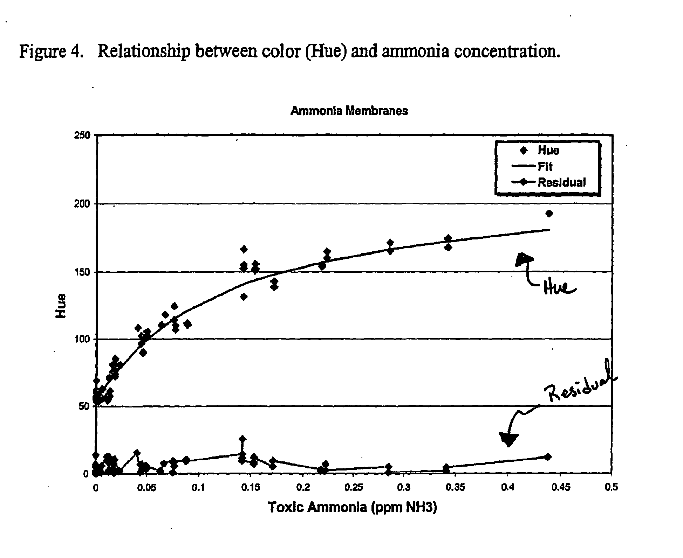

In an embodiment of the invention, the pH dye selected determines the detection range. Since the reaction responsible for the color change is analogous to the pH reaction, the pKₐ of the triphenylmethane dye may be used as a fairly accurate predictor of sensitivity. For example, the pKₐ of BTB is approximately 7.10 and demonstrates a working range of 0.0-0.50 ppm NH₃ (see FIG. 4). A pH dye with a higher pKₐ value (higher pH/NH₃ value needed to cause shift toward colored compound) would suggest that the sensitivity would be expanded to a wider range, as more NH₃ (analogous to higher local pH value) would be needed to affect the color reaction. Conversely, a pH dye with a lower pKa value would suggest the sensitivity range would be narrower than that of BTB as lower concentrations of NH₃ would affect the color change reaction. Different applications of the present invention typically necessitate monitoring of varied ammonia concentration ranges, thus making selection of the pH dye an important factor when tailoring the device for a specific application.

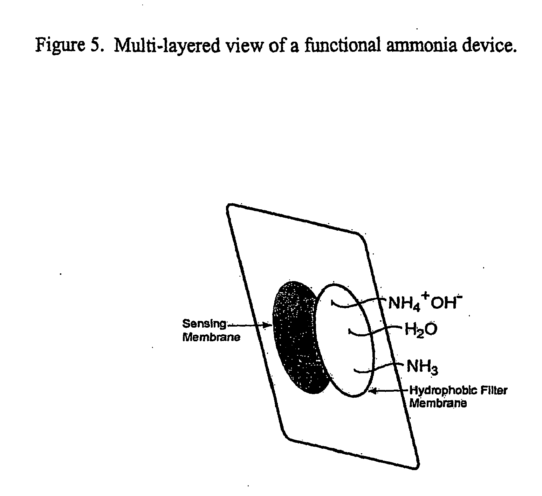

In an embodiment of the present invention, the second layer of the device is a hydrophobic filter membrane material. The hydrophobic filter material is preferably physically robust enough to withstand any physical trauma encountered in the target application so that no leaks or breaches develop. In one example, the hydrophobic material is polytetrafluoroethylene, that is TEFLON (preferably white thread seal tape, 0.0038" thickness, about 1.20 g/cm³ in density or specific gravity). In theory and in practice, however, many other hydrophobic materials would work. The function of this layer is to exclude water and substantially prevent other dissolved solids from contacting the active membrane (see FIG. 5). The hydrophobic filter layer may contact only one side of the active layer, or alternatively, may completely surround the active layer in certain embodiments of the invention.

The hydrophobic layer permits diffusion of ammonia gas through the pores so that contact between the gas and the active membrane is made. Some other examples of potential hydrophobic filter materials are polypropylene, polyvinyl chloride (PVC), polyvinylidene fluoride (PVDF), or any other material which performs the function of substantially keeping water from contacting the active membrane while allowing diffusion of ammonia gas through the pores and thus facilitating reaction of the active membrane with the dissolved ammonia. The pore size and thickness of this filter material are factors in the response time of the device, as thicker materials will inhibit diffusion to a larger extent, as will materials with smaller pore size. Nevertheless, increasing pore sizes will also lead to lower water breakthrough pressure values, i.e., the hydrophobic material will leak at lower water pressures (for example, the device would have a more limited depth performance).

Another consideration is the structural, mechanical, and chemical integrity of the hydrophobic layer. The hydrophobic material is preferably able to handle the characteristics of the target environment. For example, a material that degrades structurally in saltwater, although quite robust in freshwater, would not suit a saltwater application of this device.

The final design of the device may take different physical forms/embodiments. For example, in an embodiment of the invention the active membrane is fully enclosed between the hydrophobic filter membrane and the hydrophobic, optically transparent material. Also, the hydrophobic filter membrane can merely contact and completely cover a side of the active membrane, without fully enclosing the membrane.

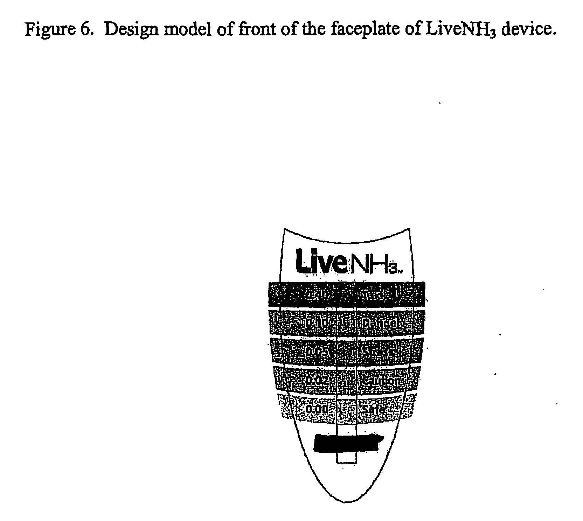

A preferred embodiment of the design of the device is illustrated in FIG. 6, whereby the invention can further include a color reference chart. Such a chart displays a plurality of colors, where each color corresponds to a reference ammonia concentration value. Reference ammonia concentration values and corresponding colors present on a color reference chart of a given invention will be selected based on the indicator dyes immobilized to the sensor membrane of the device. Appropriate colors corresponding to reference ammonia values, matched with the dyes of a device will be apparent to those skilled in the art, although preferably colors in the range of shades from yellow to green to blue will be utilized. One skilled in the art will recognize that gradual color shades between those specifically identified correspond to values between the associated and indicated ammonia concentration values.

A color reference chart is preferably positioned such that the color of the sensor membrane is quickly and easily compared to a reference color, in order to determine the ammonia concentration of the aqueous solution. The chart should be easily visible in order for the user to compare the color of the sensing layer with the colors on the reference chart and the activity required by the user for making such a comparison should be limited to directing the user's attention in the direction of the device. Typically, but not necessarily, a color reference chart is present directly on the device. For example, a color reference chart may be positioned on the optically transparent hydrophobic layer of the device and adjacent or nearby the sensor membrane, such that the user can view the sensor membrane through the transparent layer and adjacent to the color chart. In another embodiment, the color reference chart can be positioned separate from the device, but near the location of the device such that a comparison can be made by directing the user's attention to the general area of the sensor membrane.

The device can further include a structure for immersing and/or submerging the device in the aquatic or other environment. Such a structure can include a weighted body, such as a piece of metal, attached to the device to prevent the device from floating to the surface of aquatic environment. In some embodiments, the support structure is sufficient to keep the device immersed in the aquatic environment, thereby obviating the necessity of an additional structure for immersing the device. In another embodiment, a structure for immersing is coupled with the device such that the device floats freely near the surface of the aquatic environment, thereby allowing the user to visually locate and inspect the sensor membrane of the device without having to remove it from the aquatic environment, but while maintaining the sensor membrane immersed in the aquatic environment.

In another embodiment, the structure for immersing the device includes an apparatus of affixing the device to the aquatic environment container in such a manner that the entire device, including the sensor membrane, hydrophobic filter membrane, and if relevant, color reference chart, is submerged beneath the surface of the water. Such an apparatus may include suction cups, a bio-compatible and water resistant adhesive, flotation device that holds the device beneath water, a clip that attaches to a wall, side, or bank of the aquatic environment container, or a hook that also attaches to a wall of the container, but may be moved laterally without applying pressure as would be required for the clip. In this regard, the aquatic environment preferably comprises an ornamental aquarium for fish and other similar species. Various other immersion structures are possible.

Although the invention has been described with reference to the above examples, it will be understood that modifications and variations are encompassed within the spirit and scope of the invention. Accordingly, the above examples are not intended to limit the invention.

## Claims

1. A reusable device for continuously monitoring the gaseous ammonia (NH₃) concentration in a surrounding environment comprising:
a first layer comprising a sensor membrane with a indicator dye immobilized therein, wherein the indicator dye changes color corresponding to the gaseous ammonia concentration; and
a second layer comprising a hydrophobic filter membrane permeable to gaseous ammonia such that gaseous ammonia can diffuse therethrough and contact the sensor membrane, the reusable device being immersible in an aqueous environment and reusuable.

2. The device of claim 1, wherein the hydrophobic filter membrane physically separates the sensor membrane from the surrounding environment.

3. The device of claim 1, wherein the active membrane has a plurality of sides, and the active membrane is contacted on a first side by the hydrophobic filter membrane and on a second side by a hydrophobic, optically transparent material.

4. The device of claim 2, wherein the active membrane is fully enclosed between the hydrophobic filter membrane and the hydrophobic, optically transparent material.

5. The device of claim 2, wherein the hydrophobic, optically transparent material has a color reference chart with a plurality of colors displayed thereon, wherein each color corresponds to a reference gaseous ammonia concentration value.

6. The device of claim 1, wherein the sensor membrane is a polyamide membrane.

7. The device of claim 1, wherein the sensor membrane is a positively charged membrane.

8. The device of claim 1, wherein the sensor membrane is a negatively charged membrane.

9. The device of claim 1, wherein the sensor membrane is a neutral membrane.

10. The device of claim 1, wherein the indicator dye comprises one or more dyes from the group consisting of: bromophenol blue, congo red, methyl orange, resorcin blue, alizarin, methyl red, bromoceresol purple, chrophenol red, bromothymol blue, phenol red, litmus, neutral red, tumaric curcumin, and phenolphthalein.

11. The device of claim 1, wherein the sensor membrane changes color in response to gaseous ammonia concentration.

12. The device of claim 1, wherein the surrounding environment is an aqueous environment.

13. The device of claim 1, wherein the hydrophobic filter substantially prevents water, dissolved solids and other contaminants from contacting the sensor membrane.

14. The device of claim 1, wherein the hydrophobic filter substantially prevents ammonium ions from contacting the sensor membrane.

15. The device of claim 1, wherein the hydrophobic filter includes one or more compounds from the group consisting of: polytetrafluoroethylene, polypropylene, polyvinylidene fluoride, polyvinyl chloride, copolymers and blends of these compounds.

16. The device of claim 1, wherein the hydrophobic filter membrane contacts and completely covers a side of the active membrane.

17. A method for detecting the gaseous ammonia (NH₃) concentration in an aqueous environment, comprising:
providing a multi-layered reusuable device comprising a first layer having a sensor membrane with a indicator dye immobilized therein, wherein the indicator dye changes color corresponding to the gaseous ammonia concentration, and a second layer comprising a hydrophobic filter membrane permeable to gaseous ammonia such that gaseous ammonia can diffuse therethrough and contact the sensor membrane;
immersing the device in the aqueous environment; and
viewing the color change in the sensor membrane upon the gaseous ammonia contacting the sensor membrane.

18. The method of claim 17, wherein the hydrophobic filter membrane physically separates the sensor membrane from the aqueous environment.

19. The method of claim 17, wherein the reusuable device has a third layer comprising a hydrophobic, optically transparent material having a color reference chart with a plurality of colors displayed thereon, wherein each color corresponds to a reference gaseous ammonia concentration value, and further including the steps of:
viewing the color change in the sensor membrane through the hydrophobic, optically transparent material upon the gaseous ammonia contacting the sensor membrane, and
comparing the color change of the sensor membrane with the plurality of colors on the color reference chart.

20. The method of claim 19, wherein the active membrane is fully enclosed between the hydrophobic filter membrane and the hydrophobic, optically transparent material.
